# EUROPÄISCHE PATENTSCHRIFT

(11) **EP 0 775 123 B1**
(45) Veröffentlichungstag und Bekanntmachung des Hinweises auf die Patenterteilung: **30.09.1998**
(21) Anmeldenummer: 95927733.6
(22) Anmeldetag: 27.07.1995
(51) Int. Cl.: C07D 275/00, A01N 43/80

(54) **SACCHARINDERIVATE**
SACCHARIN DERIVATIVES
DERIVES DE SACCHARINE

(30) Priorität: 08.08.1994 DE 4427995
(43) Veröffentlichungstag der Anmeldung: 28.05.1997
(73) Patentinhaber: BASF AKTIENGESELLSCHAFT, 67056 Ludwigshafen (DE)
(72) Erfinder: PLATH, Peter, D-67227 Frankenthal (DE); KARDORFF, Uwe, D-68159 Mannheim (DE); VON DEYN, Wolfgang, D-67434 Neustadt (DE); ENGEL, Stefan, D-55286 Wörrstadt (DE); KAST, Jürgen, D-67459 Böhl-Iggelheim (DE); RANG, Harald, D-67122 Altrip (DE); KÖNIG, Hartmann, D-69115 Heidelberg (DE); GERBER, Matthias, D-67117 Limburgerhof (DE); WALTER, Helmut, D-67283 Obrigheim (DE); WESTPHALEN, Karl-Otto, D-67346 Speyer (DE)
(86) Internationale Anmeldenummer: EP9502973
(87) Internationale Veröffentlichungsnummer: WO9605182

(56) Entgegenhaltungen:
- EP-A- 0 243 313
- EP-A- 0 319 075
- EP-A- 0 376 072
- EP-A- 0 666 254
- US-A- 4 410 353
- US-A- 4 447 634
- US-A- 5 055 125

## Beschreibung

Gegenstand der vorliegenden Anmeldung sind Saccharinderivate der Formel I 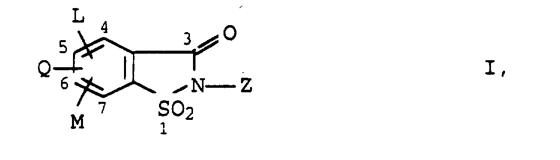 in der die Substituenten folgende Bedeutung haben:
- L,M: Wasserstoff, C₁-C₄-Alkyl, C₁-C₄-Alkoxy, C₁-C₄-Alkylthio, Chlor, Cyano, Methylsulfonyl, Nitro oder Trifluormethyl;
- Z: Wasserstoff, C₁-C₄-Alkyl, C₃-C₈-Cycloalkyl, C₃-C₆-Alkenyl, C₃-C₅-Alkinyl, C₁-C₄-Acyl, Benzyl oder Phenyl, wobei die Phenylringe jeweils gegebenenfalls durch Halogen oder C₁-C₄-Alkyl substituiert sind;
- Q: ein Rest CO-J;
- J: ein in Stellung 2 verknüpfter Cyclohexan-1,3-dionring der Formel II 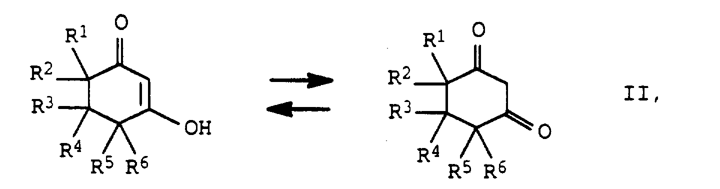 in welcher entweder R¹ bis R⁶ Wasserstoff oder Methyl bedeuten, oder, wenn R¹, R², R³, R⁵ und R⁶ Wasserstoff bedeuten, R⁴ 2-Ethylthiopropyl, Tetrahydropyranyl-3, Tetrahydropyranyl-4, Tetrahydrothiopyranyl-3 oder 1-Methylthio-cyclopropyl bedeutet,
oder, wenn R¹, R⁴, R⁵ Wasserstoff und R⁶ Methyl bedeuten, R² und R³ einen Dreiring bilden, so daß ein in Stellung 2 verknüpfter Bicyclo[4.1.0]heptanring der Formel III resultiert 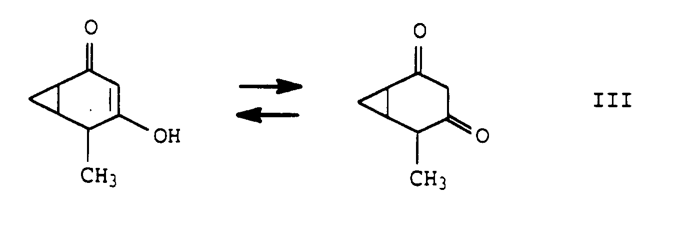 sowie landwirtschaftlich übliche Salze der Verbindungen I.

Gegenstand der Erfindung sind ferner herbizide Mittel, enthaltend die Verbindungen I sowie Verfahren zur Bekämpfung unerwünschten Pflanzenwuchses mit den Saccharinderivaten I. Darüber hinaus umfaßt die vorliegende Erfindung auch Zwischenprodukte zur Herstellung der erfindungsgemäßen Endprodukte I und Herstellungsverfahren für die Produkte I, ausgehend von den neuen Zwischenprodukten der Formeln IV und V 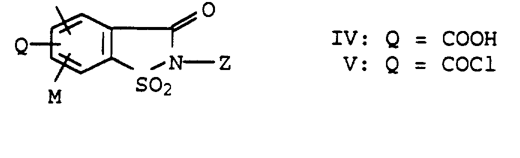 wobei die übrigen Reste L, M und Z die voranstehend genannte Bedeutung haben.

Saccharinderivate mit herbizider Wirkung sind dem Stand der Technik nicht zu entnehmen. Dagegen ist das unsubstituierte Saccharin (o-Sulfobenzoesäureimid, d.h. L, M, Q und Z in Formel I = H) seit langem als synthetischer Süßstoff bekannt. Als Süßstoff bekannt ist ferner das 4-Hydroxy-saccharin (DE-OS 3 607 343). Bekannt ist auch die Verwendung von Saccharinderivaten bei der Schädlingsbekämpfung, z.B. JP-Publikation 72/00419, 73/35457 (Fungizide) und in der Pharmazie, z.B. EP-A 594 257 und darin genannte Literaturzitate.

Herbizid wirksame 2-Aroylcyclohexandione sind Gegenstand der älteren deutschen Anmeldung P 44 03 670. Unter anderem wird eine Verknüpfung mit Saccharin und N-Alkylsaccharin erwähnt, ohne einen Herstellungsweg für diese Verbindungen anzugeben.

Heterocyclische Verbindungen mit einem eine Sulfonamidgruppe enthaltenden Ring sind als Herbizide bekannt geworden, als typischer Vertreter ist hier das Bentazon 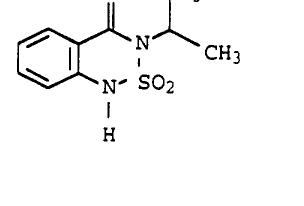 zu nennen.

Der Erfindung lag nun die Aufgabe zugrunde, neue Herbizide mit einer bislang für diese Indikation unbekannten Grundstruktur zur Verfügung zu stellen. Demgemäß wurden die eingangs definierten Verbindungen I und die Zwischenprodukte IV und V gefunden. Die Zwischenprodukte IV und deren Herstellung sind Gegenstand der zeitgleichen deutschen Anmeldung DE-A 44 27 996.

Verbindungen der Formel I erhält man dadurch, daß man Verbindungen der Formel II bzw. III mit einem Säurechlorid der Formel V acyliert und zu Saccharinderivaten der Formel I.1 umlagert:

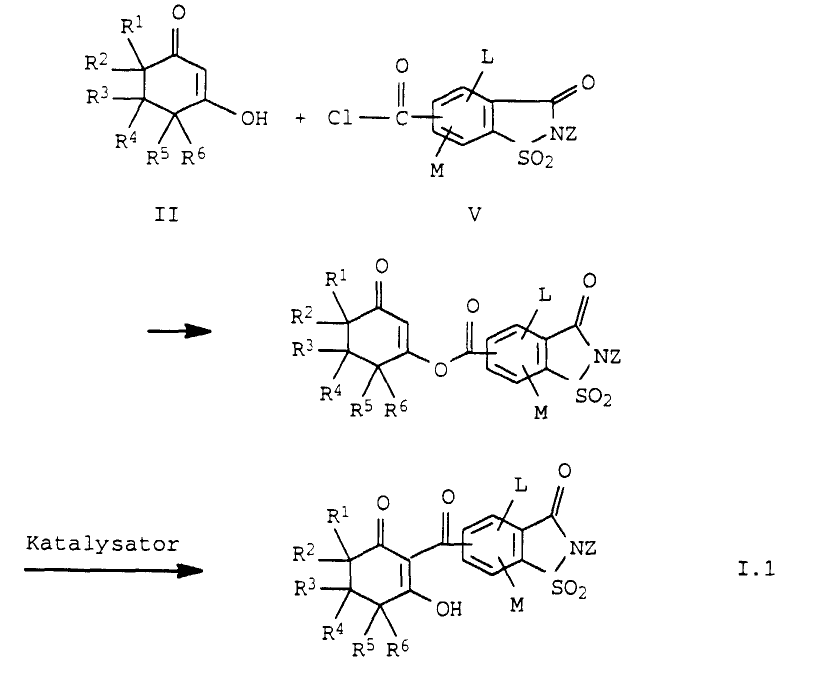

In den oben genannten Formeln haben L und M die eingangs angegebene Bedeutung und Z steht für Wasserstoff, C₁-C₄-Alkyl, C₃-C₈-Cycloalkyl, C₃-C₆-Alkenyl, C₃-C₅-Alkinyl, C₁-C₄-Acyl oder für gegebenenfalls durch Halogen oder C₁-C₄-Alkyl substituiertes Benzyl oder Phenyl.

Der erste Schritt der Reaktionsabfolge, die Acylierung, erfolgt in allgemein bekannter Weise, z. B. durch Zugabe eines Säurechlorids der Formel V zur Lösung oder Suspension eines Cyclohexan-1,3-dions II oder III in Gegenwart einer Hilfsbase. Die Reaktanden und die Hilfsbase werden dabei zweckmäßig in äquimolaren Mengen eingesetzt. Ein geringer Überschuß, z.B. 1,2 bis 1,5-Moläquivalente, bezogen auf II bzw. III, der Hilfsbase kann u.U. vorteilhaft sein.

Als Hilfsbase eignen sich tertiäre Alkylamine, Pyridin oder Alkalicarbonate. Als Lösungsmittel können z.B. Methylenchlorid, Diethylether, Toluol oder Essigsäureethylester verwendet werden.

Während der Zugabe des Säurechlorids wird die Reaktionsmischung vorzugsweise auf 0 bis 10°C gekühlt, danach wird bei einer Temperatur von 20 bis 100°C, insbesondere 25 bis 50°C gerührt, bis die Umsetzung beendet ist. Die Aufarbeitung erfolgt in üblicher Weise, z.B. wird das Reaktionsgemisch in Wasser gegossen und das Wertprodukt extrahiert, z.B. mit Methylenchlorid. Nach Trocknen der organischen Phase und Entfernung des Lösungsmittels kann der rohe Enolester ohne weitere Reinigung zur Umlagerung eingesetzt werden. Herstellungsbeispiele für Benzoyl-enolester von Cyclohexan-1,3-dione findet man z. B. in EP-A 186 118 oder US 4,780,127.

Die Umlagerung der Enolester zu den Verbindungen der Formel I.1 erfolgt zweckmäßig bei Temperaturen von 20°C bis 40°C in einem Lösungsmittel und in Gegenwart einer Hilfsbase sowie mit Hilfe einer Cyanoverbindung als Katalysator.

Als Lösungsmittel kann z.B. Acetonitril, Methylenchlorid, 1,2-Dichlorethan, Essigsäureethylester oder Toluol verwendet werden. Bevorzugtes Lösungsmittel ist Acetonitril. Als Hilfsbase eignen sich tertiäre Alkylamine, Pyridin oder Alkalicarbonate, die vorzugsweise in äquimolarer Menge oder bis zu vierfachem Überschuß, bezogen auf den Benzoylenolester, eingesetzt werden. Bevorzugte Hilfsbase ist Triethylamin in doppelter Menge.

Als Katalysator eignen sich z.B. Kaliumcyanid oder Acetoncyanhydrin, vorzugsweise in einer Menge von 1 bis 50 Molprozent, bezogen auf den Enolester. Bevorzugt setzt man Acetoncyanhydrin zu, z.B. in der Menge von 5 bis 15, insbesondere 10 Molprozent. Beispiele zur cyanidkatalysierten Umlagerung von Enolestern der Cyclohexan-1,3-dione findet man z.B. in EP-A 186 118 oder US 4,780,127.

Die Aufarbeitung erfolgt in an sich bekannter Weise. Z.B. wird das Reaktionsgemisch mit verdünnten Mineralsäuren wie 5 %iger Salzsäure oder Schwefelsäure angesäuert und mit einem organischen Lösungsmittel wie Methylenchlorid oder Essigsäureethylester extrahiert. Zur Reinigung wird der Extrakt mit kalter 5 bis 10 %iger Alkalicarbonatlösung extrahiert, wobei das Endprodukt in die wäßrige Phase übergeht. Durch Ansäuern der wäßrigen Lösung wird das Produkt der Formel I ausgefällt oder erneut mit Methylenchlorid extrahiert, getrocknet und anschließend vom Lösungsmittel befreit.

Die als Ausgangsmaterial verwendeten 1,3-Diketone der Formeln II und III sind bekannt und können nach an sich bekannten Verfahren hergestellt werden (vgl. EP-A 71 707, EP-A 142 741, EP-A 243 313, US 4 249 937 und WO 92/13821). Cyclohexandion-1,3 und Dimedon sind käufliche Verbindungen.

Die Ausgangsstoffe der Formel V werden in an sich bekannter Weise durch Umsetzung der Saccharincarbonsäurederivate IV mit Thionylchlorid hergestellt.

Saccharincarbonsäuren IV sind zum Teil bekannt (4-COOH: Zincke, Liebigs Ann. 427, 231 (1922), 5-COOH: Jacobsen, Chem. Ber. 13, 1554 (1880), 6-COOH: Weber, Chem. Ber. 25, 1740 (1892)). In der DE-OS 36 07 343 ist ferner die Herstellung der 4-Chlorsaccharin-5-carbonsäure beschrieben.

Saccharincarbonsäuren IV können auch erhalten werden, indem man man entsprechende brom- oder iodsubstituierte Saccharinderivate der Formel A1 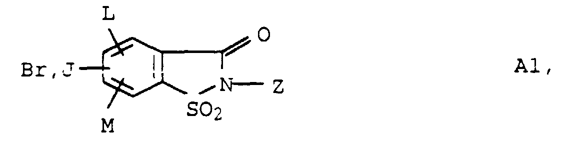 in der L, M und Z die obengenannte Bedeutung haben, oder im Falle von Z ≠ H Verbindungen der Formel A2 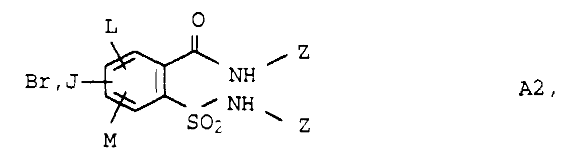 in Gegenwart eines Palladium-, Nickel-, Cobalt- oder Rhodium-Übergangsmetallkatalysators und einer Base mit Kohlenmonoxid und Wasser unter erhöhtem Druck umsetzt.

Wenn beispielsweise L für Methyl und M und Z für Wasserstoff stehen, läßt sich die Reaktionsfolge wie folgt darstellen:

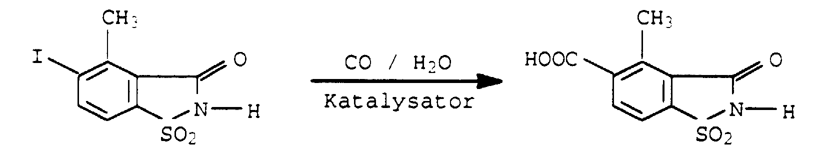

Die Katalysatoren Nickel, Cobalt, Rhodium und insbesondere Palladium können metallisch oder in Form üblicher Salze wie in Form von Halogenverbindungen, z.B. PdCl₂, RhCl₃·H₂O, Acetaten, z.B. Pd(OAc)₂, Cyaniden usw. in den bekannten Wertigkeitsstufen vorliegen. Ferner können Metallkomplexe mit tertiären Phosphinen, Metallalkylcarbonyle, Metallcarbonyle, z.B. CO₂(CO)₈, Ni(CO)₄, Metallcarbonyl-Komplexe mit tertiären Phosphinen, z.B. (PPh₃)₂Ni(CO)₂, oder mit tertiären Phosphinen komplexierte Übergangsmetallsalze vorliegen. Die letztgenannte Ausführungsform ist insbesondere im Fall von Palladium als Katalysator bevorzugt. Dabei ist die Art der Phosphinliganden breit variabel. Beispielsweise lassen sie sich durch folgende Formeln wiedergeben: 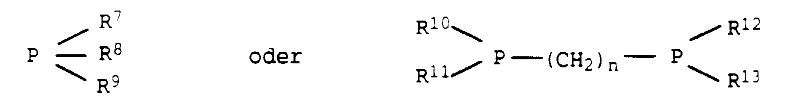 wobei n die Zahlen 1, 2, 3 oder 4 bedeutet und die Reste R⁷ bis R¹³ für niedermolekulares Alkyl, z.B. C₁-C₆-Alkyl, Aryl, C₁-C₄-Alkyl-aryl, z.B. Benzyl, Phenethyl oder Aryloxy stehen. Aryl ist z.B. Naphthyl, Anthryl und vorzugsweise gegebenenfalls substituiertes Phenyl, wobei man hinsichtlich der Substituenten nur auf deren Inertheit gegenüber der Carboxylierungsreaktion zu achten hat, ansonsten können sie breit variiert werden und umfassen alle inerten C-organischen Reste wie C₁-C₆-Alkylreste, z.B. Methyl, Carboxylreste wie COOH, COOM (M ist z.B. ein Alkali-, Erdalkalimetall oder Ammoniumsalz), oder C-organische Reste über Sauerstoff gebunden wie C₁-C₆-Alkoxyreste.

Die Herstellung der Phosphinkomplexe kann in an sich bekannter Weise, z.B. wie in den eingangs genannten Dokumenten beschrieben, erfolgen. Beispielsweise geht man von üblichen kommerziell erwerblichen Metallsalzen wie PdCl₂ oder Pd(OCOCH₃)₂ aus und fügt das Phosphin z.B. P(C₆H₅)₃, P(n-C₄H₉)₃, PCH₃(C₆H₅)₂, 1,2-Bis(diphenylphosphino)ethan hinzu.

Die Menge an Phosphin, bezogen auf das Übergangsmetall, beträgt üblicherweise 0 bis 20, insbesondere 0,1 bis 10 Moläquivalente, besonders bevorzugt 1 bis 5 Moläquivalente.

Die Menge an Übergangsmetall ist nicht kritisch. Natürlich wird man aus Kostengründen eher eine geringe Menge, z.B. von 0,1 bis 10 Mol.-%, insbesondere 1 bis 5 Mol.-%, bezogen auf den Ausgangsstoff Al bzw. A2 verwenden.

Zur Herstellung der Saccharincarbonsäuren IV führt man die Umsetzung mit Kohlenmonoxid und mindestens äquimolaren Mengen an Wasser, bezogen auf die Ausgangsstoffe A1 bzw. A2 durch. Der Reaktionspartner Wasser kann gleichzeitig auch als Lösungsmittel dienen, d.h. die maximale Menge ist nicht kritisch.

Es kann aber auch je nach Art der Ausgangsstoffe und der verwendeten Katalysatoren von Vorteil sein, anstelle des Reaktionspartners ein anderes inertes Lösungsmittel oder die für die Carboxylierung verwendete Base als Lösungsmittel zu verwenden.

Als inerte Lösungsmittel kommen für Carboxylierungsreaktionen übliche Lösungsmittel wie Kohlenwasserstoffe, z.B. Toluol, Xylol, Hexan, Pentan, Cyclohexan, Ether z.B. Methyl-tert.butylether, Tetrahydrofuran, Dioxan, Dimethoxyethan, substituierte Amide wie Dimethylformamid, persubstituierte Harnstoffe wie Tetra-C₁-C₄-alkylharnstoffe oder Nitrile wie Benzonitril oder Acetonitril in Betracht.

In einer bevorzugten Ausführungsform des Verfahrens verwendet man einen der Reaktionspartner, insbesondere die Base, im Überschuß, so daß kein zusätzliches Lösungsmittel erforderlich ist.

Für das Verfahren geeignete Basen sind alle inerten Basen, die den bei der Umsetzung freiwerdenden Jodwasserstoff bzw. Bromwasserstoff zu binden vermögen. Beispielsweise sind hier tertiäre Amine wie tert.-Alkylamine, z.B. Trialkylamine wie Triethylamin, cyclische Amine wie N-Methylpiperidin oder N,N'-Dimethylpiperazin, Pyridin, Alkali- oder -hydrogencarbonate, oder tetraalkylsubstituierte Harnstoffderivate wie Tetra-C₁-C₄-alkylharnstoff, z.B. Tetramethylharnstoff, zu nennen.

Die Menge an Base ist nicht kritisch, üblicherweise werden 1 bis 10, insbesondere 1 bis 5 Mol verwendet. Bei gleichzeitiger Verwendung der Base als Lösungsmittel, wird die Menge in der Regel so bemessen, daß die Reaktionspartner gelöst sind, wobei man aus Praktikabilitätsgründen unnötig hohe Überschüsse vermeidet, um Kosten zu sparen, kleine Reaktionsgefäße einsetzen zu können und den Reaktionspartnern maximalen Kontakt zu gewährleisten.

Während der Umsetzung wird der Kohlenmonoxiddruck so eingestellt, daß immer ein Überschuß an CO, bezogen auf A1 bzw. A2 vorliegt. Vorzugsweise liegt der Kohlenmonoxiddruck bei Raumtemperatur bei 1 bis 250 bar, insbesondere 5 bis 150 bar CO.

Die Carbonylierung wird in der Regel bei Temperaturen von 20 bis 250°C, insbesondere bei 30 bis 150°C kontinuierlich oder diskontinuierlich durchgeführt. Bei diskontinuierlichem Betrieb wird zweckmäßigerweise zur Aufrechterhaltung eines konstanten Druckes kontinuierlich Kohlenmonoxid auf das Umsetzungsgemisch aufgepreßt.

Aus dem anfallenden Reaktionsgemisch werden die Produkte in üblicher Weise, z.B. durch Destillation isoliert.

Die für die Umsetzung benötigten Ausgangsstoffe Al bzw. A2 sind bekannt oder können in an sich bekannter Weise hergestellt werden. Sie können entweder durch Permanganatoxidation von iodsubstituierten 2-Methylbenzolsulfonamiden oder durch Sandmeyer-Reaktion aus Aminosacchariden erhalten werden. Aminosaccharine werden nach bekannten Methoden durch Reduktion von Nitrosacchariden erhalten, die ihrerseits entweder bekannt sind (Kastle, Amer. Chem. Journal 11, 184 (1889) oder DRP 551423 (1930) oder in literaturbekannter Weise aus geeigneten Nitrobenzolderivaten (Liebigs Ann. 669, 85 (1963)) oder Benzolsulfonamiden synthetisiert werden.

Darüber hinaus können sie analog den Herstellvorschriften aus den Beispielen 1 bis 12 erhalten werden.

Im Hinblick auf die bestimmungsgemäße Verwendung sind Saccharinderivate der Formel I bevorzugt, in der die Reste L bzw. M für Wasserstoff, Methyl, Methoxy, Methylthio, Chlor, Cyano, Methylsulfonyl, Nitro oder Trifluormethyl stehen. Ferner sind Saccharinderivate I bevorzugt, in denen einer der Reste L oder M oder beide Reste L und M ungleich Wasserstoff sind.

In Formel I bedeuten die Reste R¹ bis R⁶ entweder alle Wasserstoff oder ein, zwei oder drei Reste Methyl und die übrigen Reste Wasserstoff. Die Restekombination R³ + R⁴ = CH₃ und R¹, R², R⁵, R⁶ = H ist neben dem unsubstituierten Cyclus (R¹ bis R⁶ = H) besonders bevorzugt.

Der Rest Z steht besonders bevorzugt für einen der genannten C-organischen Reste, insbesondere für Methyl, Ethyl, Propargyl, Acetyl oder Phenyl.

Insbesondere bevorzugte Wirkstoffe sind den Tabellen 1 bis 7 zu entnehmen.

Die Verbindungen I können in Form ihrer landwirtschaftlich brauchbaren Salze vorliegen, wobei es auf die Art des Salzes im allgemeinen nicht ankommt. Üblicherweise werden die Salze von solchen Basen in Betracht kommen, welche die herbizide Wirkung von I nicht negativ beeinträchtigen.

Als basische Salze eignen sich besonders diejenigen der Alkalimetalle, vorzugsweise die Natrium- und Kaliumsalze, die der Erdalkalimetalle, vorzugsweise Calcium-, Magnesium-, und Bariumsalze und die der Übergangsmetalle, vorzugsweise Mangan-, Kupfer-, Zink- und Eisensalze sowie die Ammoniumsalze, die ein bis drei C₁-C₄-Alkyl, Hydroxy-C₁-C₄-alkylsubstituenten und/oder einen Phenyl- oder Benzylsubstituenten tragen können, vorzugsweise Diisopropylammonium-, Tetramethylammonium-, Tetrabutylammonium-, Trimethylbenzylammonium-, und Trimethyl-(2-hydroxyethyl)ammoniumsalze, die Phosphoniumsalze, die Sulfoniumsalze, vorzugsweise Tri-(C₁-C₄-)alkylsulfoniumsalze, und die Sulfoxoniumsalze, vorzugsweise Tri-(C₁-C₄-)alkylsulfoxoniumsalze.

Die Verbindungen I die sie enthaltenden herbiziden Mittel sowie deren umweltverträgliche Salze von beispielsweise Alkalimetallen, Erdalkalimetallen oder Ammoniak und Aminen bzw. die sie enthaltenden herbiziden Mittel können in Kulturen wie Weizen, Reis, Mais, Soja und Baumwolle Unkräuter und Schadgräser sehr gut bekämpfen, ohne die Kulturpflanzen nennenswert zu schädigen. Dieser Effekt tritt vor allem bei niedrigen Aufwandmengen auf.

Unter Berücksichtigung der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber lassen sich die Verbindungen I auch in Kulturen, die durch Züchtung und/oder mittels gentechnischer Methoden gegen die Wirkung von I oder anderen Herbiziden weitgehend resistent gemacht wurden, einsetzen.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Oldispersionen kommen im wesentlichen in Betracht: Mineralölfraktionen von mittlerem bis hohem Siedepunkt wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline oder deren Derivate, alkylierte Benzole und deren Derivate, Alkohole wie Methanol, Ethanol, Propanol, Butanol und Cyclohexanol, Ketone wie Cyclohexanon oder stark polare Lösungsmittel, z.B. Amine wie N-Methylpyrrolidon, oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.-%, vorzugsweise zwischen 0,5 und 90 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. 20 Gewichtsteile der Verbindung Nr. 2.08 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
II. 20 Gewichtsteile der Verbindung Nr. 2.08 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
III. 20 Gewichtsteile des Wirkstoffs Nr. 2.08 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. 2.08 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile des Wirkstoffs Nr. 2.08 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.08 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Zur Verbreiterung des Wirkungsspektrums und zur Erzielung synergistischer Effekte können die Saccharinderivate mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

### Herstellungsbeispiele

A) Herstellung der Ausgangsstoffe
   1. 2-Methyl-6-acetaminobenzoesäure
      Zu einer Lösung von 24,8 g (0,62 Mol) NaOH in 500 ml Wasser gibt man 90,6 g (0,6 Mol) 6-Methylanthranilsäure und tropft dann 63,4 g (0,62 Mol) Acetanhydrid zu. Nach einer Stunde Nachrühren wird unter Kühlung mit konz. HCl auf pH 3 angesäuert, der ausfallende Niederschlag wird abgesaugt, mit Wasser gewaschen und bei 50°C i.Vak. getrocknet.
      Ausbeute: 107 g (0,55 Mol) = 92 % d.Th., Fp.: 189 - 190°C
   2. 2-Methyl-3-nitro-6-acetaminobenzoesäure
      Man legt 271 ml 98 proz. Salpetersäure bei - 5°C vor und trägt portionsweise 106 g (0,55 Mol) der unter 1. hergestellten 2-Methyl-6-acetaminobenzoesäure ein. Nach einer Stunde Nachrühren bei 10°C wird das Reaktionsgemisch in eine Mischung aus 540 g Eis und 270 ml Wasser gegossen. Der ausgefallene Niederschlag wird abgesaugt, mit Wasser gewaschen und bei 50°C i.Vak. getrocknet.
      Ausbeute: 75,6 g (0,317 Mol) = 58 % d.Th.,
      Fp.: 190 - 191°C
      Aus dem Filtrat scheidet sich nach längerem Stehen das in 3-Stellung nitrierte Isomere ab:
      Ausbeute: 21,3 g (0.089 Mol) = 16 % d.Th.,
      Fp.: 180 - 182°C
   3. 2-Methyl-3-nitro-6-aminobenzoesäure
      Man legt 450 ml 2-N NaOH vor und fügt 75,6 g (0,317 Mol) 2-Methyl-3-nitro-6-acetaminobenzoesäure zu. Anschließend erwärmt man die Reaktionsmischung auf 95°C und läßt eine Stunde bei dieser Temperatur rühren. Nach Abkühlung auf 10°C wird durch Zugabe von 425 ml 2-N HCl angesäuert, saugt den ausfallenden Niederschlag ab, wäscht mit Wasser und trocknet bei 50°C i.Vak..
      Ausbeute: 50,7 g (0,258 Mol) = 82 % d.Th.,
      Fp.: 183 - 184°C
      Beim Versuch, diesen Acetylrest im Sauren abzuspalten, würde Decarboxylierung erfolgen.
   4. 2-Methyl-3-nitro-6-aminobenzoesäuremethylester
      Man löst 49,7 g (0,253 Mol) 2-Methyl-3-nitro-6-aminobenzoesäure in 380 ml Aceton und fügt 43 g (0,51 Mol) Natriumhydrogencarbonat zu. Anschließend wird zum Sieden erhitzt, bis die CO₂-Entwicklung abgeschlossen ist. Zu der so erhaltenen Suspension des Natriumsalzes von 2-Methyl-3-nitro-6-aminobenzoesäure tropft man anschließend bei Siedetemperatur des Acetons 35,3 g (0,28 Mol) Dimethylsulfat im Laufe von zwei Stunden zu, refluxiert anschließend noch drei Stunden und läßt dann abkühlen. Nach Eingießen der Reaktionsmischung in 1,8 1 Wasser wird mit Methylenchlorid extrahiert. Nach Trocknen der organischen Phase wird eingeengt. Der erhaltene Feststoff ist für die Folgeumsetzung genügend rein (NMR).
      Ausbeute: 50 g (0,238 Mol) = 94 % d.Th., Fp.: 92 - 94°C
   5. 2-Methoxycarbonyl-3-methyl-4-nitro-benzolsulfonsäurechlorid
      Man löst unter Erwärmen 58,5 g (0,278 Mol) 2-Methyl-3-nitro-6-amino-benzoesäure-methylester in 280 ml Eisessig und gießt diese Lösung b° 15 bis 20°C in 85 ml konz. HCl. Dann tropft man bei 5 bis 10°C eine Lösung von 19,3 g (0,28 Mol) Natriumnitrit in 60 ml Wasser zu und läßt 30 min bei 5°C nachrühren. Anschließend tropft man diese Diazoniumsalzlösung in eine Lösung von 374 g SO₂ in 750 ml Eisessig, die 14 g CuCl₂ (in 30 ml Wasser gelöst) enthält. Nach Beendigung der Stickstoffentwicklung wird noch 15 min nachgerührt und dann in 1,4 1 Eiswasser gegossen. Das Sulfonsäurechlorid wird durch Extraktion mit 1,2 1 Methylenchlorid abgetrennt. Nach Trocknen und Einengen der organischen Phase erhält man 73 g (0,25 Mol) (= 90 % d.Th.) eines Öls, das nach NMR (in CDCl₃) reines 2-Methoxycarbonyl-3-methyl-4-nitrobenzolsulfonsaurechlorid ist.
   6. 4-Methyl-5-nitrosaccharin
      Man legt 104 ml 25 proz. Ammoniaklösung vor, fügt 100 ml Wasser zu und tropft dann bei 10°C eine Lösung von 48,7 g (0,166 Mol) 2-Methoxycarbonyl-3-methyl-4-nitro-benzolsulfonsäurechlorid in 70 ml Tetrahydrofuran zu. Nach drei Stunden Rühren bei 25°C wird Wasser und THF weitgehend entfernt und der verbleibende Rückstand mit Essigester verrührt, abgesaugt und mit Essigester gewaschen. Nach dem Trocknen i.Vak. erhält man 34 g (0,131 Mol) = 79 % d.Th. eines weißen Feststoffs mit Fp.: 312°C (Zers.)
   7. 2,4-Dimethyl-5-nitrosaccharin
      Diese Substanz kann durch nachträgliche Methylierung des unter 6. erhaltenen Saccharins mit Dimethylsulfat in Gegenwart von NaOH hergestellt werden.
   8. 3-Methyl-4-nitro-2-(N'-methyl)carboxamido-N-methylbenzolsulfonamid
      Man gießt 50 ml Wasser in 50 ml 40 proz. Methylamin-Lösung und tropft bei 10°C eine Lösung von 24,3 g (83 mMol) 2-Methoxycarbonyl-3-methyl-4-nitro-benzolsulfonsäurechlorid in 35 ml THF zu. Nach einer Stunde Rühren bei 25°C werden alle flüchtigen Bestandteile entfernt. Der Rückstand wird mit Essigester extrahiert, die organische Phase mit Wasser gewaschen, getrocknet und eingeengt. Der verbleibende Rückstand kristallisiert nach längerem Stehen.
      Ausbeute: 10,3 g (40 mMol = 48 % d.Th.),
      Fp.: 125 - 126°C, nach Umkristallisation aus Essigester Fp.: 144 - 145°C.
   9. 4-Methyl-5-aminosaccharin
      Man löst unter Erwärmen auf 45°C 33,6 g (0,13 Mol) 4-Methyl-5-nitrosaccharin in 1,2 l Wasser und fügt 5 g Pd/C (10 proz. auf Aktivkohle) zu. Anschließend leitet man unter heftigem Rühren Wasserstoffgas ein (drucklose Hydrierung). Im Laufe von 4,5 Stunden werden 9 l H₂ aufgenommen. Nach Abkühlen auf 25°C wird vom Katalysator abfiltriert, auf 200 ml Volumen eingeengt und dann auf pH 1 angesäuert. Der ausgefallene Niederschlag wird abgesaugt, mit Wasser gewaschen und bei 50°C i.Vak. getrocknet. Man erhält 23,4 g (0,11 mol = 85 % d.Th.) eines weißen Feststoffs mit Fp.: 272 - 273°C
   10. 4-Methyl-5-iodsaccharin
      Man legt eine Mischung von 205 ml Eisessig, 160 ml Wasser und 40 ml konz. HCl vor und trägt unter Rühren 23,4 g (0,11 Mol) 4-Methyl-5-amino-saccharin bei 15 - 20°C ein. Zu der entstandenen Suspension tropft man bei 5 - 10°C 7,9 g (0,115 Mol) Natriumnitrit und läßt 30 min bei 5°C nachrühren. Das als Suspension vorliegende Diazoniumsalz wird dann portionsweise in eine auf 50°C erwärmte Lösung von 19,1 g (0,115 Mol) Kaliumiodid in 170 ml Wasser getropft, wobei Stickstoff entsteht. Nach Abkühlen auf Raumtemperatur wird das ausgefallene Produkt durch Absaugen isoliert, mit Wasser gewaschen und bei 50°C i.Vak. getrocknet. Man erhält 32,5 g (0,1 Mol = 91 % d.Th.) eines Feststoffs mit Fp.: 257 - 258°C. Eine Verbrennungsanalyse ergab einen Iodgehalt von 38,5 % (Theorie 39,3 %).
      Das Produkt ist für die Folgeumsetzungen genügend rein.
   11. 4-Amino-3-methyl-2-(N'-methyl)carboxamido-N-methylbenzolsulfonamid
      Analog dem bei Beispiel 9 beschriebenen Vorgehen wird das nach Beispiel 8 erhaltene 3-Methyl-4-nitro-2-(N'-methyl)carboxamido-N-methyl-benzolsulfonamid drucklos hydriert. Man erhält die Titelverbindung mit Fp. 217 - 218°C in 93 % Ausbeute.
   12. 3-Methyl-4-iod-2-(N'-methyl)carboxamido-N-methylbenzolsulfonamid
      Nach dem unter Beispiel 10 beschriebenen Vorgehen wird die voranstehende Verbindung diazotiert und durch Umsetzung mit KI zu der Titelverbindung umgesetzt. Ausbeute: 95 % d.Th., Fp.: 60 - 62°C
B) Herstellung der Zwischenprodukte IV
   13. 4-Methylsaccharin-5-carbonsäure
      6,4 g (0,002 mol) 4-Methyl-5-iod-saccharin werden in 70 ml Tetramethylharnstoff und 30 ml Wasser 0,7 g gelöst, mit Bis(triphenylphosphin)palladiumchlorid versetzt und das Gemisch in einem 300-ml-Autoklaven auf 100°C erhitzt und 36 h bei einem Druck von 100 bar Kohlenmonoxid gerührt.
      Zur Aufarbeitung filtriert man und entfernt Wasser und Tetramethylharnstoff destillativ im Hochvakuum. Der Rückstand wird in Methyltert.-butylether (MTBE) aufgenommen, mit NaHCO₃-Lsg, extrahiert und nach dem Ansäuern mit HCl wieder mit MTBE extrahiert. Nach dem Einengen erhält man 2,8 g 4-Methyl-saccharin-5-carbonsäure (58 % d.Th.).
      ¹H-NMR (DMSO, 400,1 MHz): 2,85 (3H, s); 8,05 (1H, d); 8,2 (1H, d);
      ¹³C-NMR (DMSO, 100, 6 MHz): 167,4 (CO); 161,3 (CO); 141,6 (quart. C); 139,7 (quart. C); 138,7 (quart. C); 135,6 (CH); 125,4 (quart. C); 118,5 (CH); 15,4 (CH₃).
   14. 2,4-Dimethylsaccharin-5-carbonsäure
      7,3 g (0,02 mol) 3-Methyl-4-iod-2-(N'-methyl)carboxamido-N-methylbenzol- sulfonamid werden zusammen mit 0,69 g Bis(triphenylphosphin)palladiumchlorid, 30 ml Wasser und 70 ml Tetramethylharnstoff in einem 300-ml-Autoklaven vorgelegt, das Gemisch auf 100°C erhitzt und 36 h bei einem Druck von 100 bar Kohlenmonoxid gerührt.
      Nach Aufarbeitung (wie in Beispiel 13 beschrieben) erhält man 4,1 g der Titelverbindung (0,014 mol = 72 % d.Th.).
      ¹H-NMR (DMSO, 400,1 MHz): 2,9 (3H, s); 3,15 (3H, s); 8,2 (2H, 2d); 14,0 (1H, s)
      ¹³C-NMR (DMSO, 100,6 MHz): 167,3 (CO); 158,6 (CO); 139,7 (quart. C); 139,1 (quart. C); 138,9 (quart. C); 135,5 (CH); 124,6 (quart. C); 119,0 (CH); 22,9 (CH3); 15,6 (CH₃).
   15. 2,4-Dimethyl-saccharin-5-carbonsäurechlorid
      Man suspendiert 3,8 g (14,9 mMol) 2,4-Dimethylsaccharin-5-carbonsäure in 100 ml Toluol, erwärmt auf 80°C und tropft 3,5 g (29,8 mMol) Thionylchlorid zu. Nach zwei Stunden Refluxieren wird heiß dekantiert und das Reaktionsgemisch eingeengt.
   Ausbeute: 74 % d.Th., Fp.: 149 - 150°C.
   In analoger Weise können die in Tabelle 8 zusammengestellten Saccharincarbonsäurechloride V erhalten werden.
C) Umsetzung der Zwischenprodukte V zu den herbizid wirksamen Endprodukten I.
   16. Acylierung von Cyclohexandion 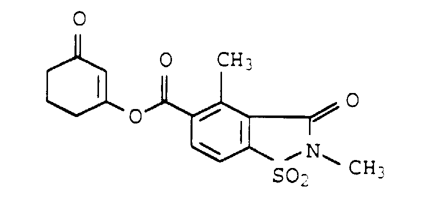
      Zu einer Suspension von 1,23 g (10,9 mMol) Cyclohexandion-1,3 in 50 ml Methylenchlorid gießt man 1,21 g (12 mMol) Triethylamin und tropft anschließend bei 25°C eine Lösung von 3 g (10,9 mMol) 2,4-Dimethylcaccharin-5-carbonsäure-chlorid in 60 ml Methylenchlorid zu. Anschließend wird 7 Stunden bei 40°C gerührt. Nach dem Abkühlen werden 60 ml Wasser zugegossen, in einem Scheidetrichter die Methylenchloridphase abgetrennt und über Magnesiumsulfat getrocknet. Der nach dem Abziehen des Lösungsmittels verbleibende amorphe Rückstand (2,5 g) ist der Enolester, der ohne weitere Reinigung in der nächsten Stufe umgelagert wird.
   17. Umlagerung zum Endprodukt I 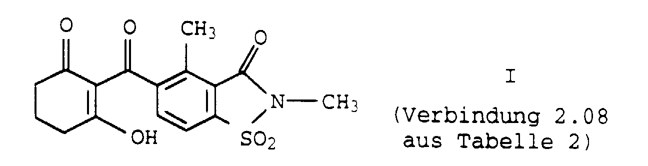
      2,5 g (7,2 mMol) des voranstehenden Enolesters werden in 80 ml Acetonitril gelöst, mit 3,5 ml Triethylamin und dann mit 0,33 g (4 mMol) Acetoncyanhydrin versetzt und 16 h gerührt. Danach werden 24,5 g 5 proz. HCl zugegeben und das Reaktionsgemisch mit 100 ml Methylenchlorid extrahiert. Anschließend wird die organische Phase mit 5 proz. Kaliumcarbonatlösung extrahiert, wobei das Produkt in die wäßrige Phase übergeht. Durch Ansäuern der alkalisch-wäßrigen Lösung mit konz. HCl wird ein gummiartiger Feststoff ausgefällt, der nach Anreiben mit Diisopropylether auskristallisiert. Nach Waschen mit Petrolether wird i.Vak. getrocknet.
      Ausbeute: 0,88 g (35 % d.Th.)

In analoger Weise werden die nachstehenden Verbindungen erhalten:

Die in den Tabellen 1 bis 8 für einen Substituenten genannten Gruppen stellen außerdem für sich betrachtet - unabhängig von der speziellen Kombination mit anderen Substituenten, in der sie genannt sind - eine besonders bevorzugte Definition des betreffenden Substituenten dar.

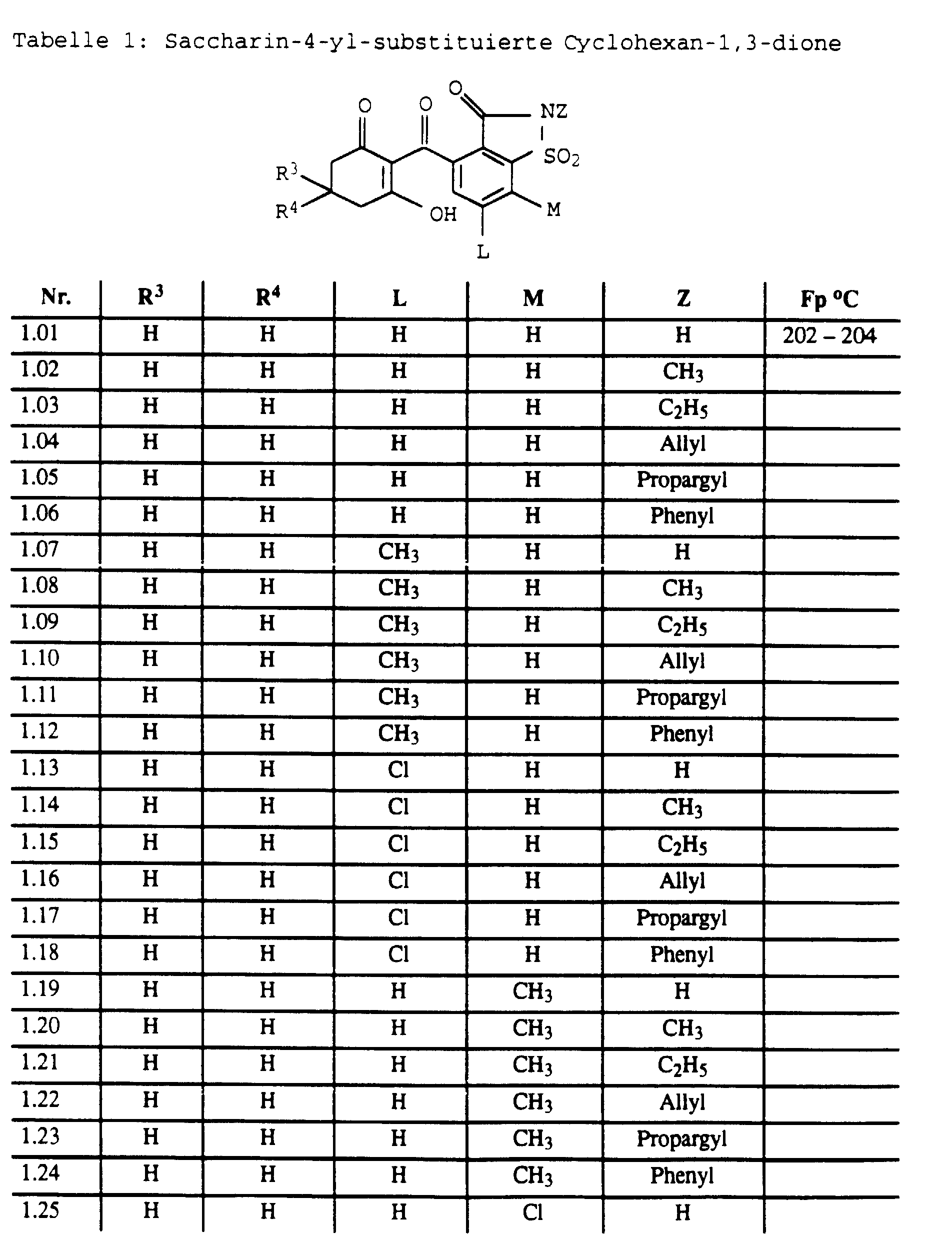

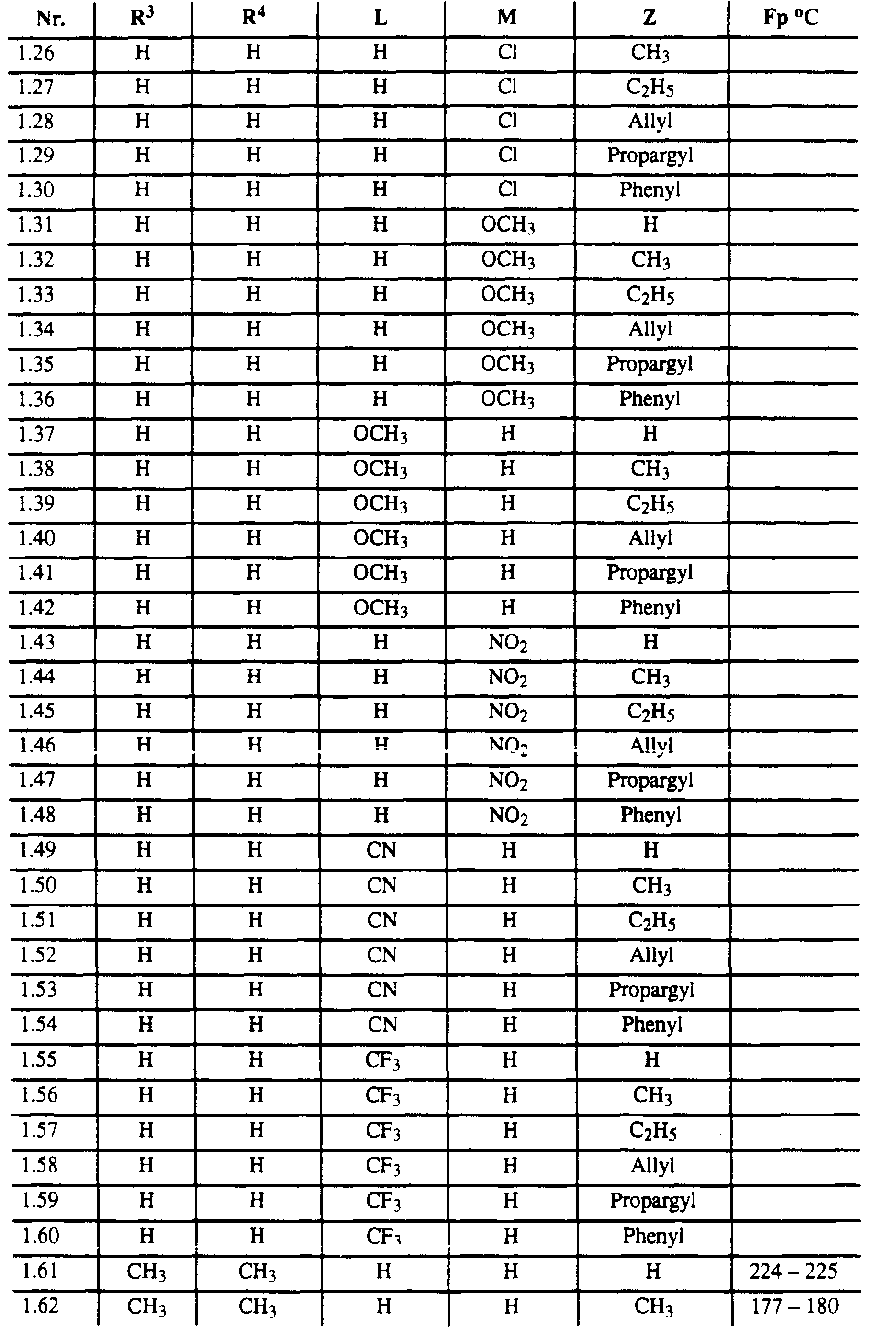

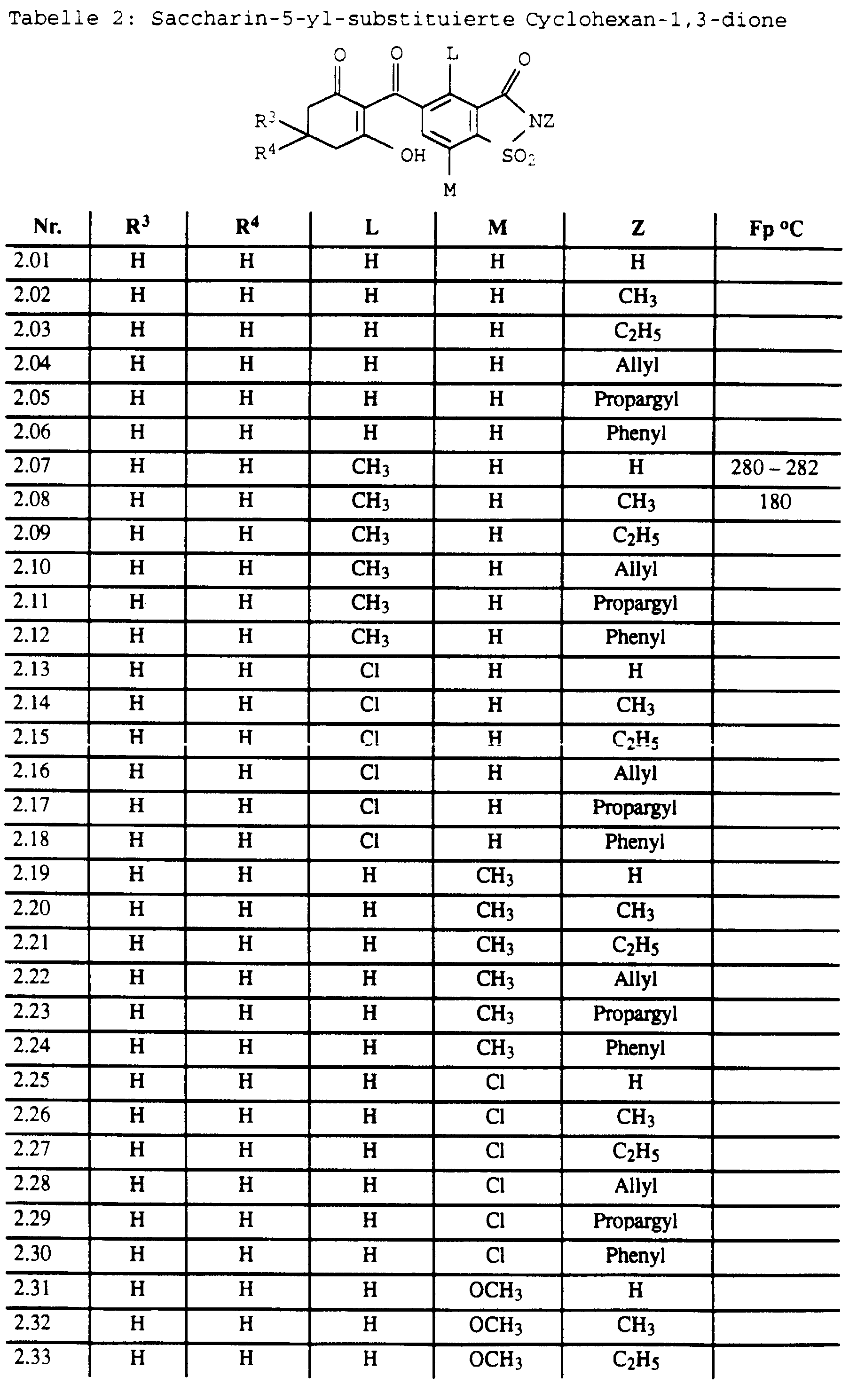

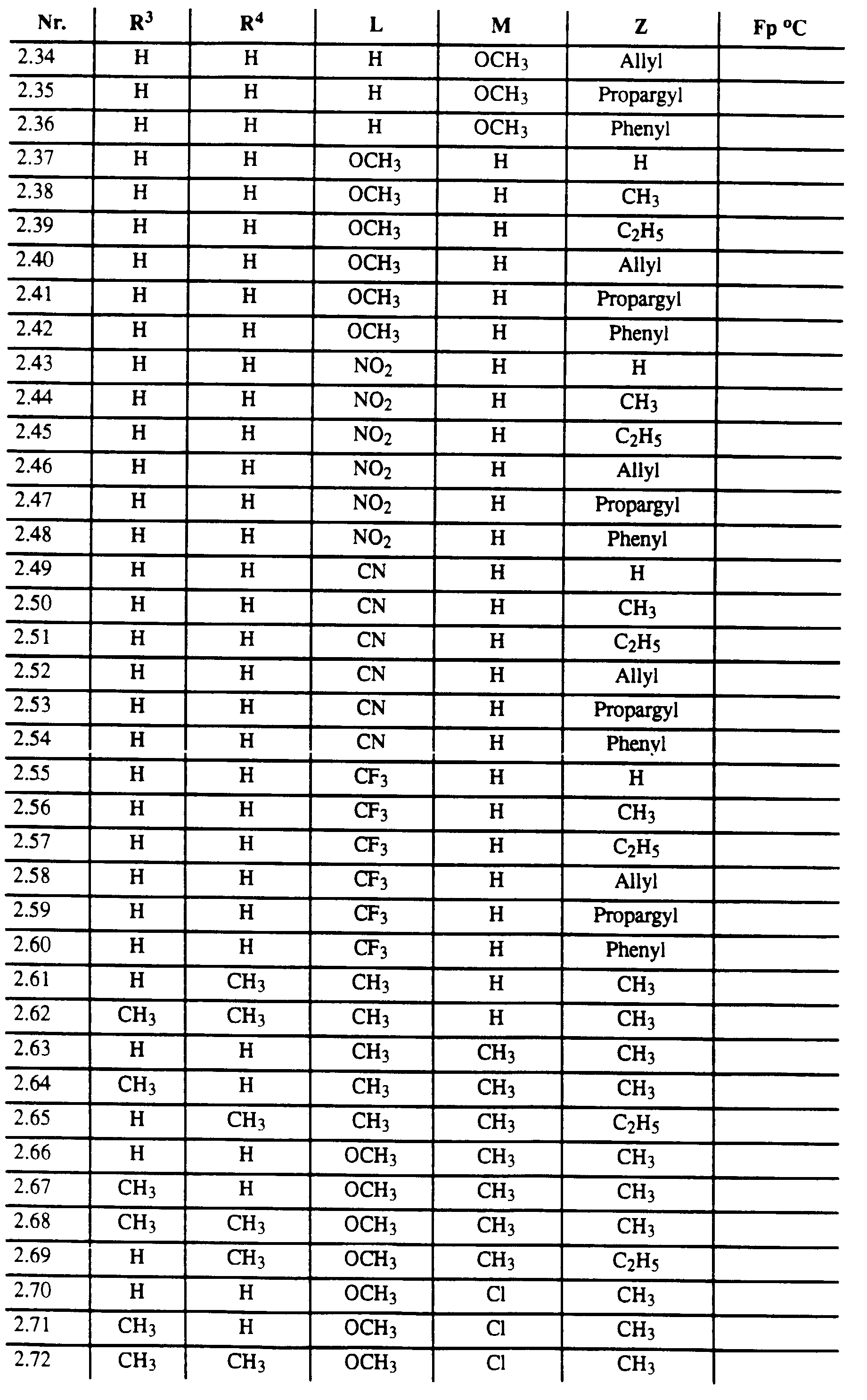

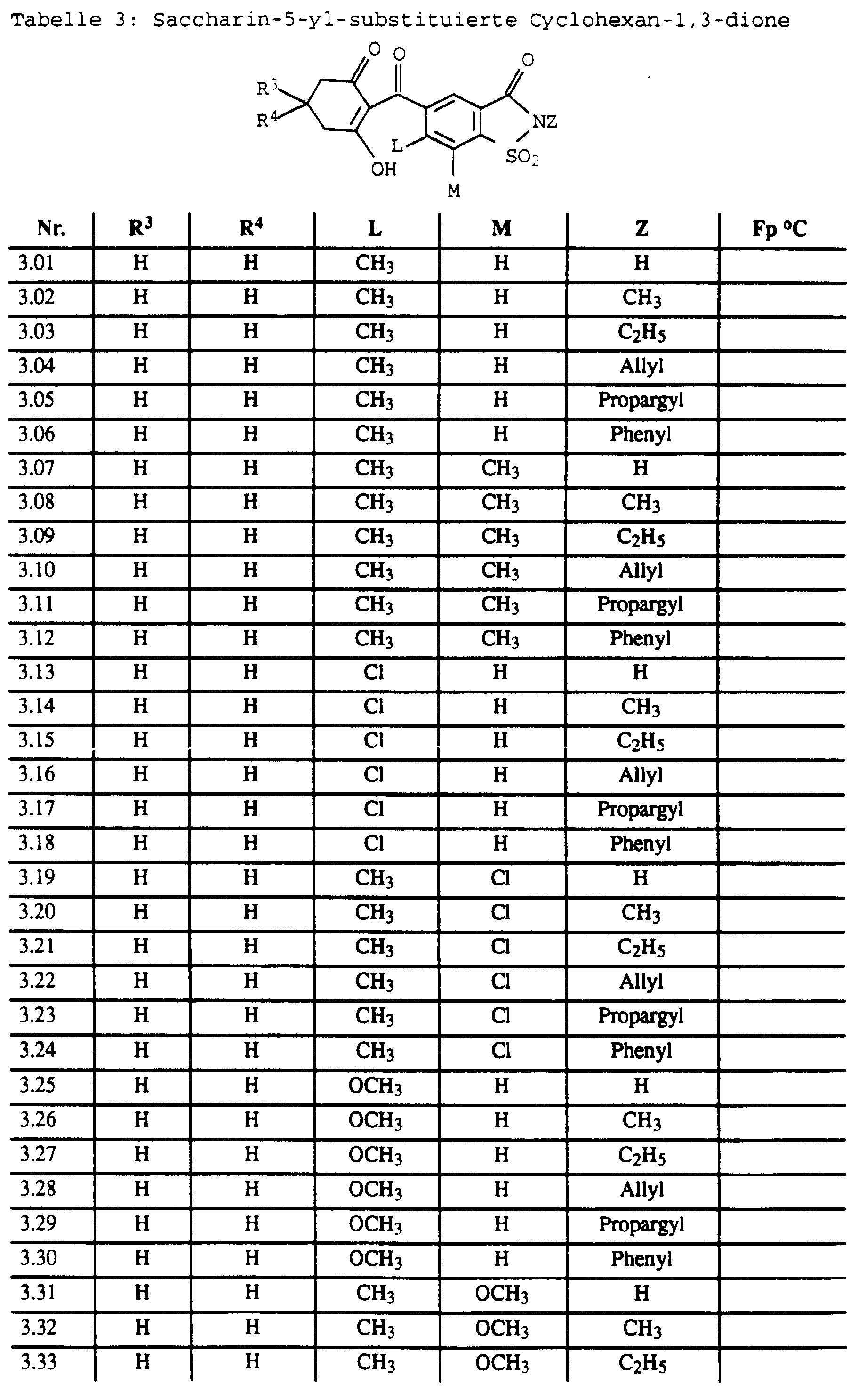

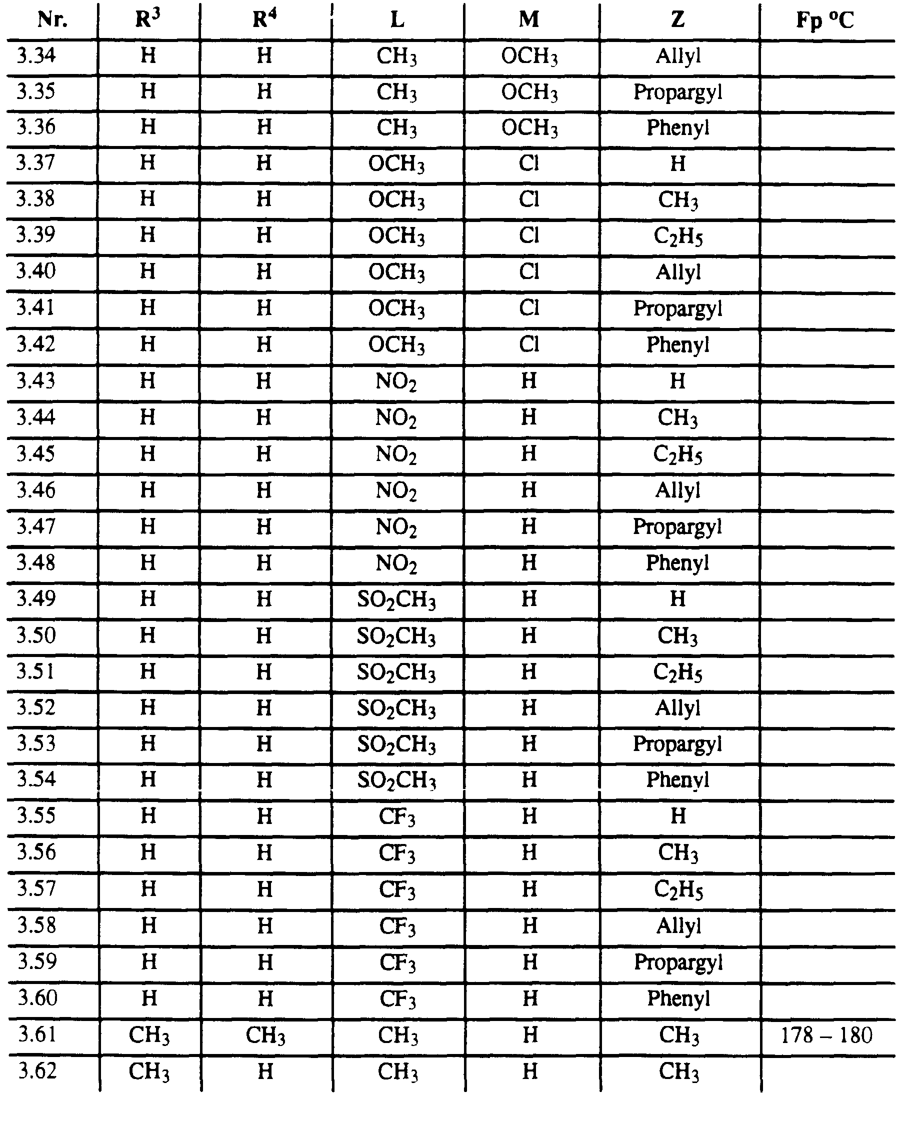

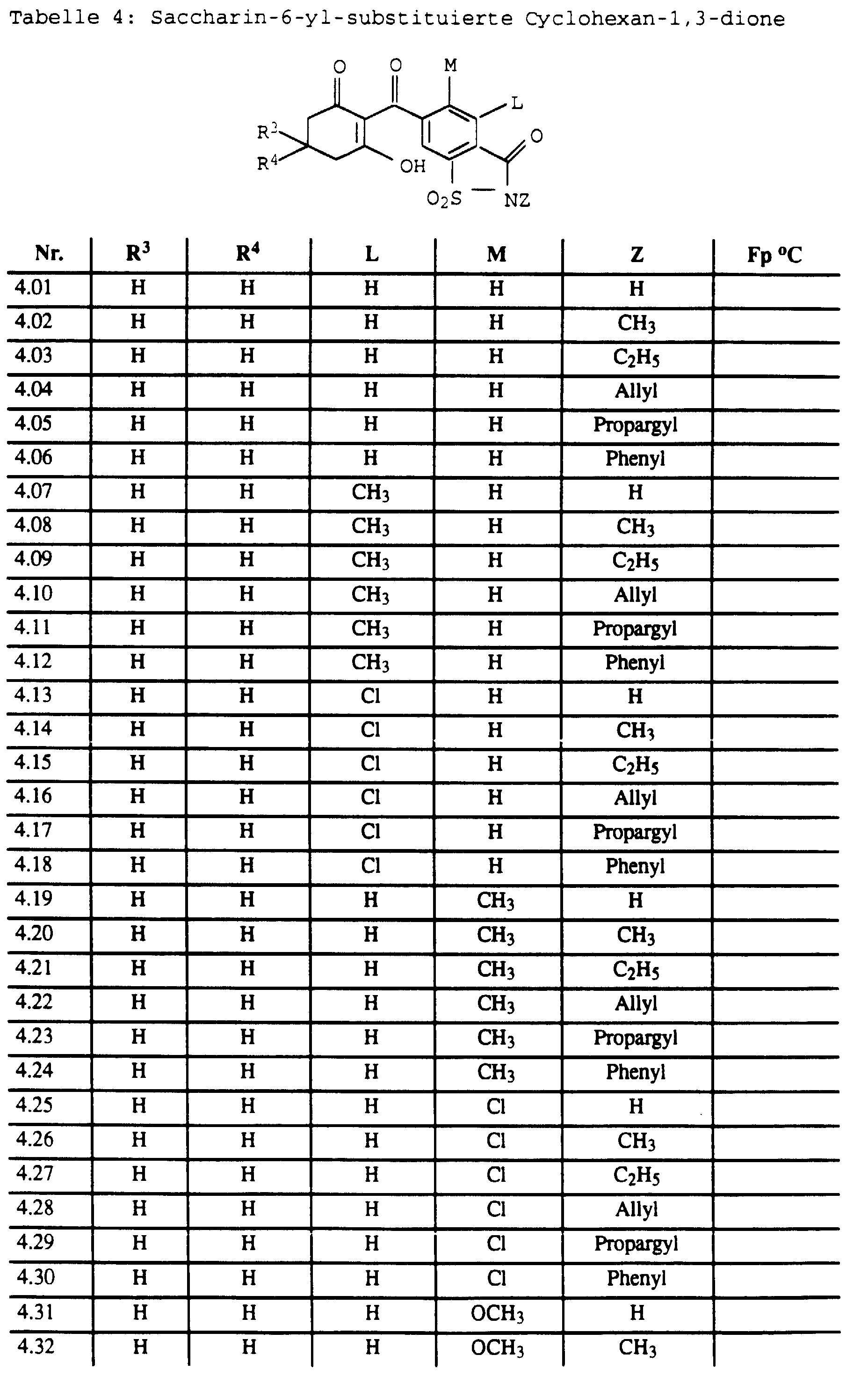

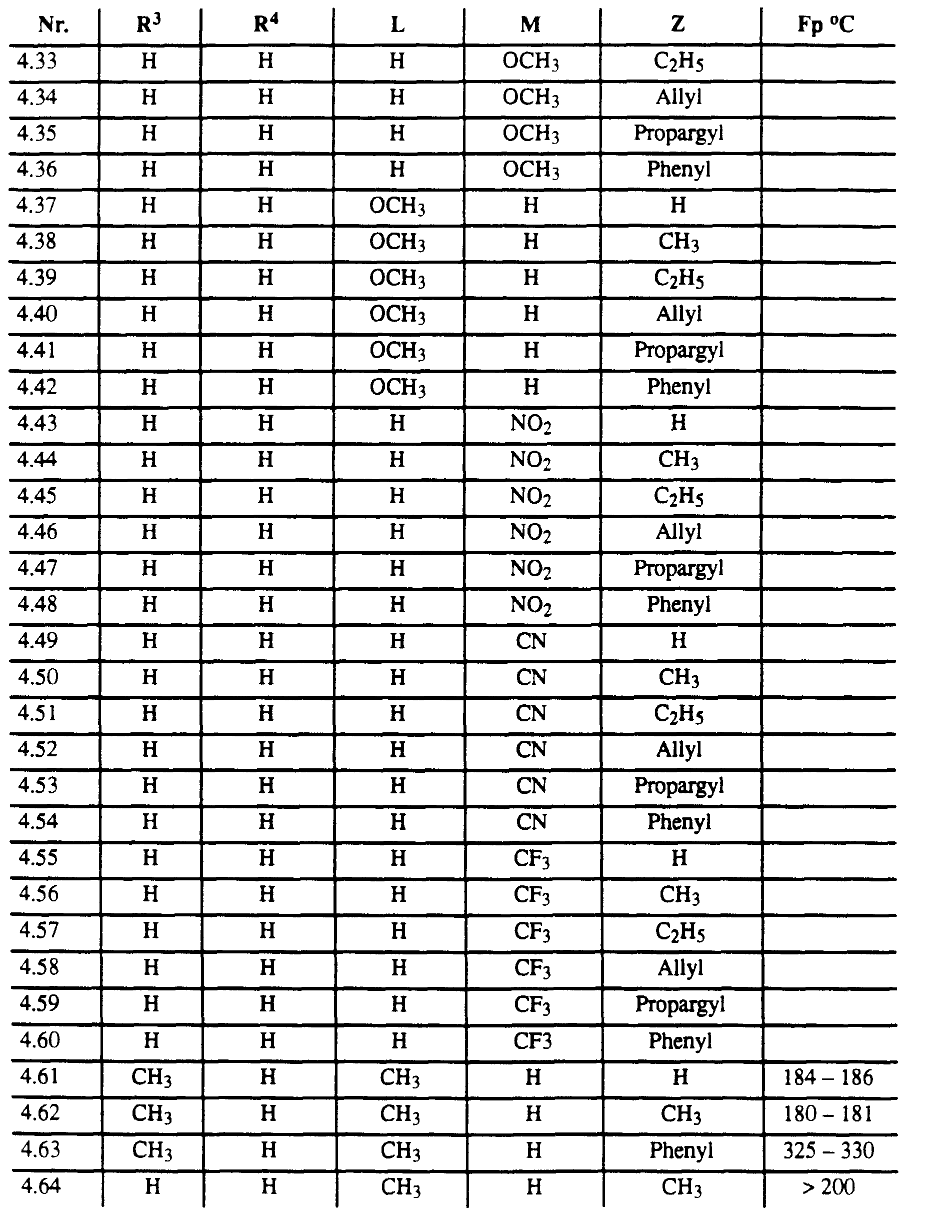

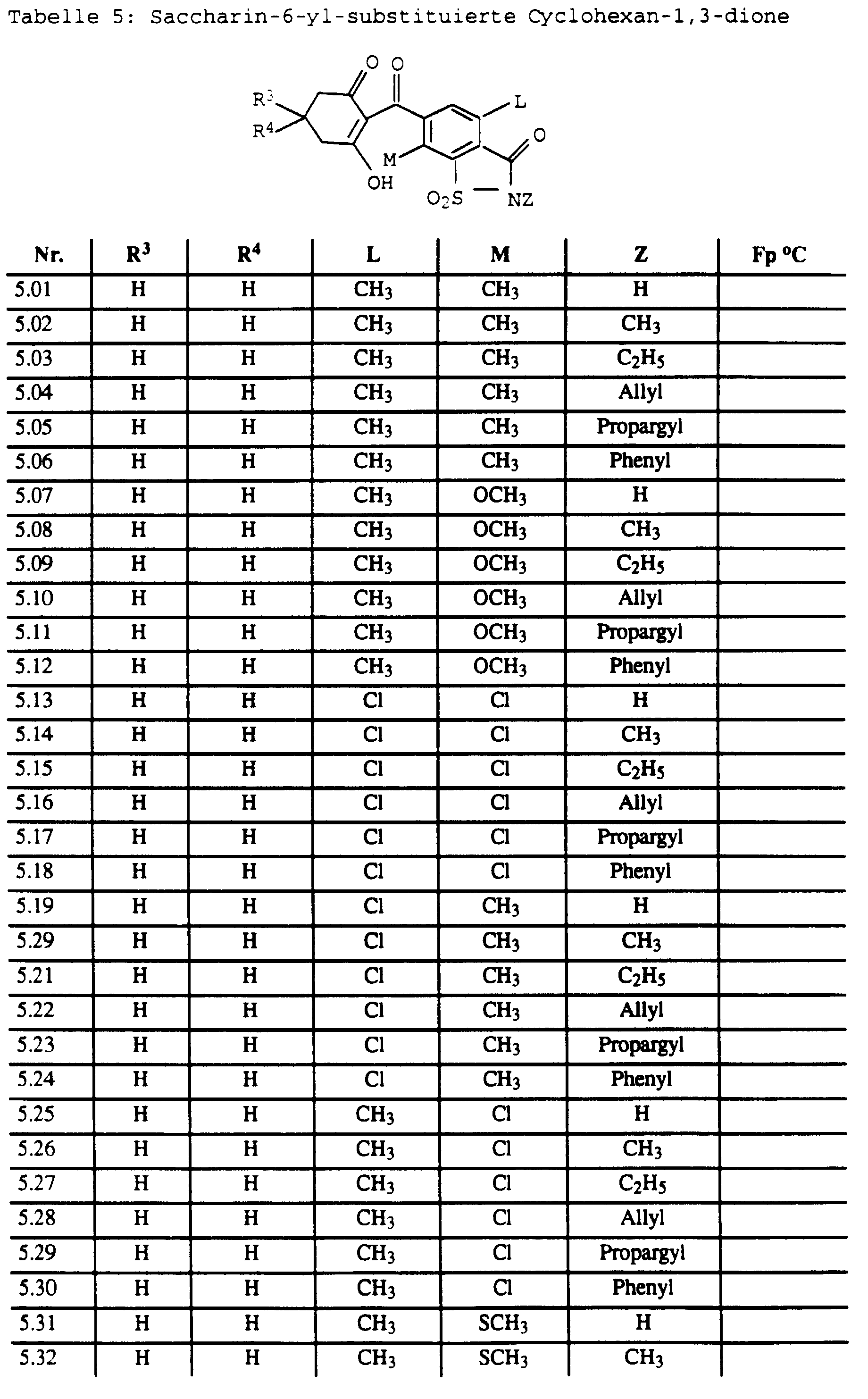

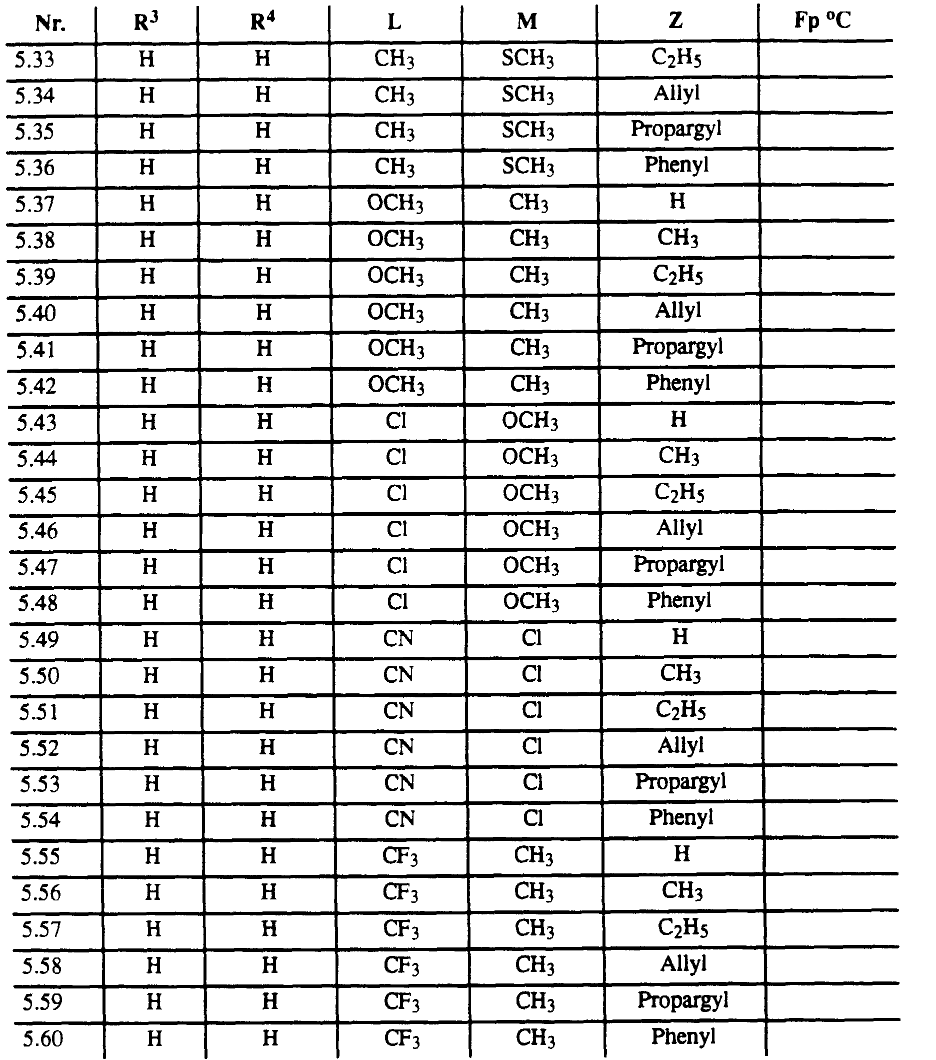

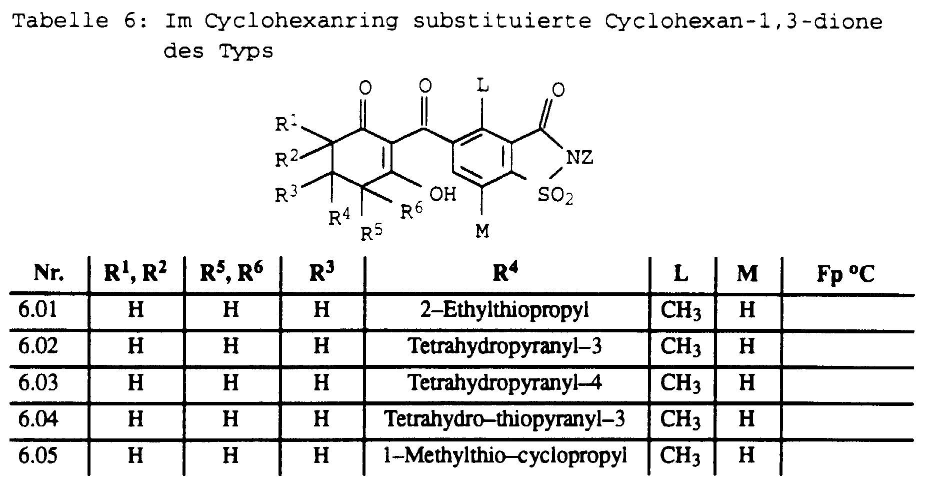

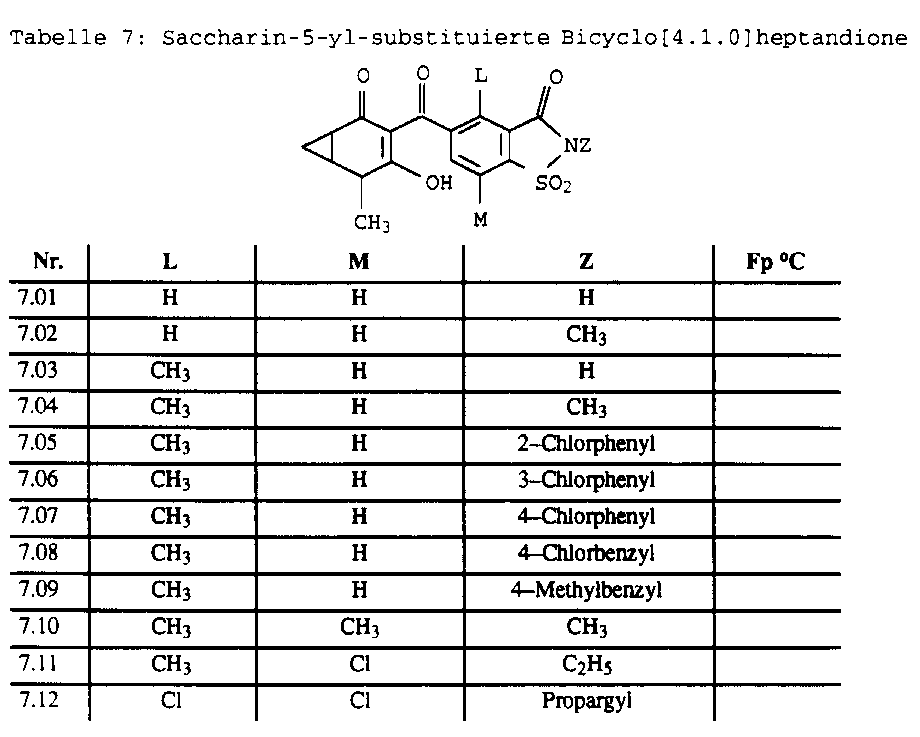

### Anwendungsbeispiele

Die herbizide Wirkung der Saccharinderivate der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie werden erst als Keimpflanzen getrennt angezogen und einige Tage vor der Behandlung in die Versuchsgefäße verpflanzt. Die Aufwandmenge für die Nachauflaufbehandlung betrug 0,5 bzw. 0,25 kg/ha a.S.

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Alopecurus myosuroides | Ackerfuchsschwanz | blackgrass |
| Avena fatua | Flughafer | wild oats |
| Zea mays | Mais | Indian corn |

Bei einer Aufwandmenge von 0,5 bzw. 0,25 kg/ha a.S. lassen sich mit der Verbindung aus Beispiel 2.08 unerwünschte Pflanzen im Nachauflaufverfahren sehr gut bekämpfen.

## Patentansprüche

1. Saccharinderivate der Formel I 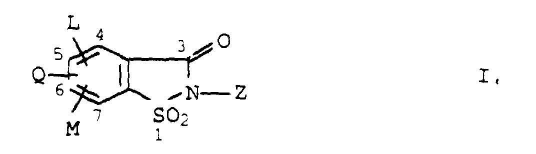 in der die Substituenten folgende Bedeutung haben:
L,M Wasserstoff, C₁-C₄-Alkyl, C₁-C₄-Alkoxy, C₁-C₄-Alkylthio, Chlor, Cyano, Methylsulfonyl, Nitro oder Trifluormethyl;
Z Wasserstoff, C₁-C₄-Alkyl, C₃-C₈-Cycloalkyl, C₃-C₆-Alkenyl, C₃-C₅-Alkinyl, C₁-C₄-Acyl, Benzyl oder Phenyl, wobei die Phenylringe jeweils gegebenenfalls durch Halogen oder C₁-C₄-Alkyl substituiert sind;
Q ein Rest CO-J;
J ein in Stellung 2 verknüpfter Cyclohexan-1,3-dionring der Formel II 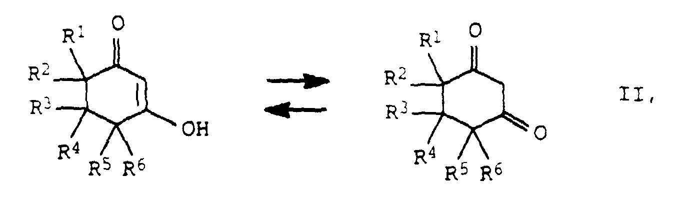 in welcher entweder R¹ bis R⁶ Wasserstoff oder Methyl bedeuten, oder, wenn R¹, R², R³, R⁵ und R⁶ Wasserstoff bedeuten, R⁴ 2-Ethylthiopropyl, Tetrahydropyranyl-3, Tetrahydropyranyl-4, Tetrahydrothiopyranyl-3 oder l-Methylthio-cyclopropyl bedeutet,
oder, wenn R¹, R⁴, R⁵ Wasserstoff und R⁶ Methyl bedeuten, R² und R³ einen Dreiring bilden, so daß ein in Stellung 2 verknüpfter Bicyclo[4,1.0]heptanring der Formel III resultiert 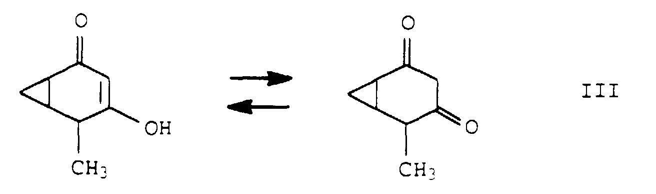 sowie landwirtschaftlich übliche Salze der Verbindungen I.

2. Saccharinderivate der Formel I gemäß Anspruch 1, in der L für C₁-C₄-Alkyl, C₁-C₄-Alkoxy, C₁-C₄-Alkylthio, Chlor, Cyano, Methylsulfonyl, Nitro oder Trifluormethyl und M für Wasserstoff oder für C₁-C₄-Alkyl, C₁-C₄-Alkoxy, C₁-C₄-Alkylthio, Chlor, Cyano, Methylsulfonyl, Nitro oder Trifluormethyl steht.

3. Saccharinderivate der Formel I gemäß den Ansprüchen 1 und 2, in der die Reste L bzw. M für Wasserstoff, Methyl, Methoxy, Methylthio, Chlor, Cyano, Methylsulfonyl, Nitro oder Trifluormethyl stehen.

4. Herbizides Mittel, enthaltend mindestens ein Saccharinderivat der Formel I gemäß Anspruch 1 und übliche inerte Zusatzstoffe.

5. Verfahren zur Bekämpfung unerwünschten Pflanzenwuchses, dadurch gekennzeichnet, daß man eine herbizid wirksame Menge eines Saccharinderivates der Formel I gemäß Anspruch 1 auf die Pflanzen oder deren Lebensraum einwirken läßt.

6. Verfahren zur Herstellung der Verbindungen der Formel I gemäß Anspruch 1, dadurch gekennzeichnet, daß man die jeweiligen Ausgangsstoffe der Formeln II oder III 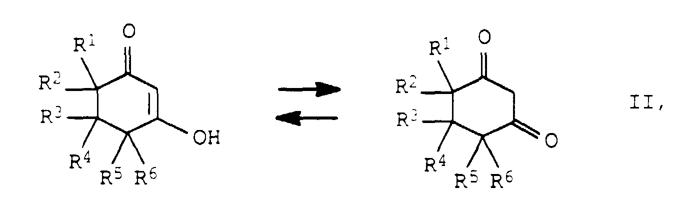 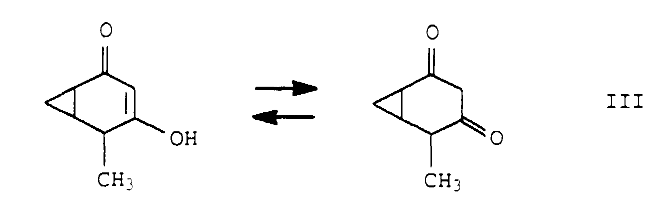 mit einem Säurechlorid der Formel V 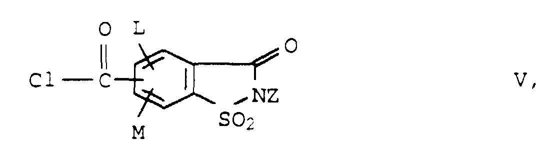 wobei L, M und Z die in Anspruch 1 genannte Bedeutung haben, acyliert und das Acylierungsprodukt in Gegenwart eines Katalysators zu den Verbindungen I umlagert.

7. Saccharinderivate der Formel V 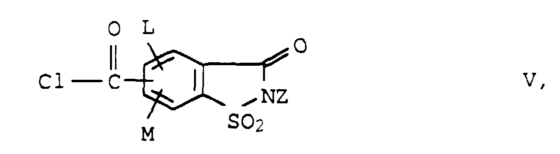 in der L, M und Z die folgende Bedeutung haben:
L,M Wasserstoff, C₁-C₄-Alkyl, C₁-C₄-Alkoxy, C₁-C₄-Alkylthio,Chlor, Cyano, Methylsulfonyl, Nitro oder Trifluormethyl;
Z Wasserstoff, C₁-C₄-Alkyl, C₃-C₈-Cycloalkyl, C₃-C₆-Alkenyl, C₃-C₅-Alkinyl, C₁-C₄-Acyl, Benzyl oder Phenyl, jeweils im Phenylring gegebenenfalls durch Halogen oder C₁-C₄-Alkyl substituiert.

## Claims

1. A saccharin derivative of the formula I 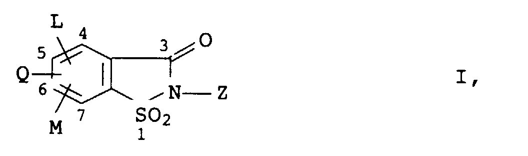 where the substituents have the following meanings:
L and M are hydrogen, C₁-C₄-alkyl, C₁-C₄-alkoxy, C₁-C₄-alkylthio, chlorine, cyano, methylsulfonyl, nitro or trifluoromethyl;
Z is hydrogen, C₁-C₄-alkyl, C₃-C₈-cycloalkyl, C₃-C₆-alkenyl, C₃-C₅-alkynyl, C₁-C₄-acyl, benzyl or phenyl, the phenyl rings in each case being unsubstituted or substituted by halogen or C₁-C₄-alkyl;
Q is a radical CO-J;
J is a 2-linked cyclohexane-1,3-dione ring of the formula II 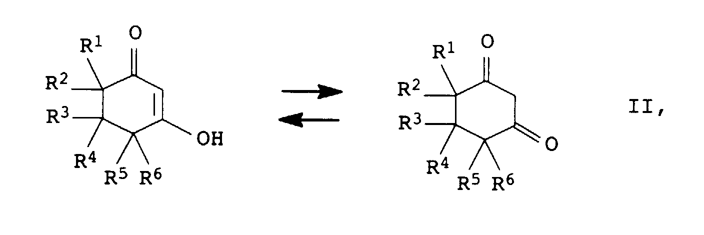
where either R¹ to R⁶ are hydrogen or methyl, or, if R¹, R², R³, R⁵ and R⁶ are hydrogen, R⁴ is 2-ethylthiopropyl, tetrahydropyran-3-yl, tetrahydropyran-4-yl, tetrahydrothiopyran-3-yl or 1-methylthiocyclopropyl,
or, if R¹, R⁴ and R⁵ are hydrogen and R⁶ is methyl, R² and R³ form a three-membered ring such that a 2-linked bicyclo-[4.1.0]heptane ring of the formula III results 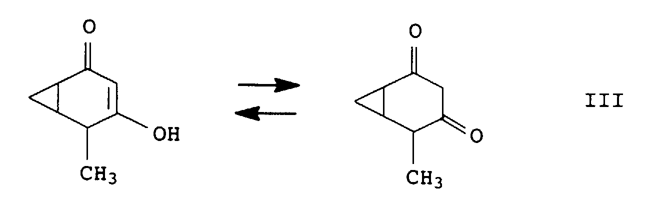 and agriculturally customary salts of the compounds I.

2. A saccharin derivative of the formula I as claimed in claim 1, where L is C₁-C₄-alkyl, C₁-C₄-alkoxy, C₁-C₄-alkylthio, chlorine, cyano, methylsulfonyl, nitro or trifluoromethyl and M is hydrogen or C₁-C₄-alkyl, C₁-C₄-alkoxy, C₁-C₄-alkylthio, chlorine, cyano, methylsulfonyl, nitro or trifluoromethyl.

3. A saccharin derivative of the formula I as claimed in claims 1 and 2, where the radicals L and M are hydrogen, methyl, methoxy, methylthio, chlorine, cyano, methylsulfonyl, nitro or trifluoromethyl.

4. A herbicidal composition, containing at least one saccharin derivative of the formula I as claimed in claim 1 and customary inert additives.

5. A method of controlling undesired plant growth, which comprises allowing a herbicidally active amount of a saccharin derivative of the formula I as claimed in claim 1 to act on the plants or their habitat.

6. A process for preparing the compounds of the formula I as claimed in claim 1, which comprises acylating the respective starting substances of the formulae II or III 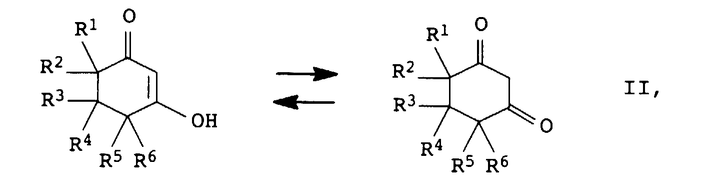 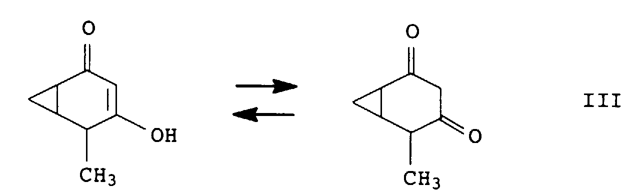 with an acid chloride of the formula V 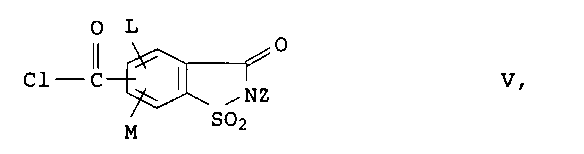 where L, M and Z have the meanings mentioned in claim 1, and rearranging the acylation product to the compounds I in the presence of a catalyst.

7. A saccharin derivative of the formula V 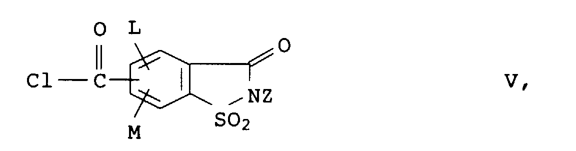 where L, M and Z have the following meanings:
L and M are hydrogen, C₁-C₄-alkyl, C₁-C₄-alkoxy, C₁-C₄-alkylthio, chlorine, cyano, methylsulfonyl, nitro or trifluoromethyl;
Z is hydrogen, C₁-C₄-alkyl, C₃-C₈-cycloalkyl, C₃-C₆-alkenyl, C₃-C₅-alkynyl, C₁-C₄-acyl, benzyl or phenyl, in each case unsubstituted or substituted by halogen or C₁-C₄-alkyl in the phenyl ring.

## Revendications

1. Dérivés de la saccharine, de formule I 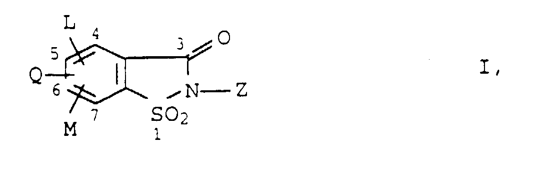 dans laquelle les symboles ont les significations suivantes :
L, M : l'hydrogène, des groupes alkyle en C1-C4, alcoxy en C1-C4, alkylthio en C1-C4, le chlore, des groupes cyano, méthylsulfonyle, nitro ou trifluorométhyle ;
Z : l'hydrogène, un groupe alkyle en C1-C4, cycloalkyle en C3-C8, alcényle en C3-C6, alcynyle en C3-C5, acyle en C1-C4, benzyle ou phényle, les cycles phényle pouvant dans chaque cas être substitués le cas échéant par des halogènes ou des groupes alkyle en C1-C4 ;
Q : un groupe CO-J ;
J : un cycle cyclohexane-1,3-dione relié en position 2, qui répond à la formule II 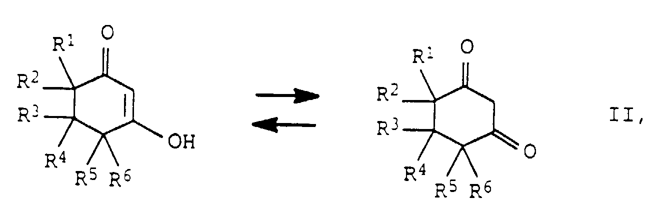 dans laquelle R¹ à R⁶ représentent l'hydrogène ou des groupes méthyle, ou bien, lorsque R¹, R², R³, R⁵, R⁶ représentent l'hydrogène, R⁴ représente un groupe 2-éthylthio-propyle, tétrahydropyrannyl-3, tétrahydropyrannyl-4, tétrahydrothiopyrannyl-3 ou l-méthylthiocyclopropyle, ou bien, lorsque R¹, R⁴, R⁵ représentent l'hydrogène et R⁶ un groupe méthyle, R² et R³ forment un cycle à trois chaînons résultant en la formation d'un cycle bicyclo[4.1.0] heptane relié en position 2 et répondant à la formule III 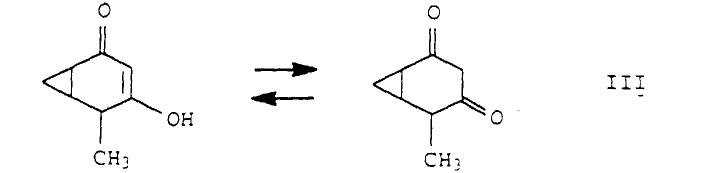 et les sels des composés I convenant pour les applications agricoles.

2. Dérivés de la saccharine de formule I de la revendication 1, dans laquelle L représente un groupe alkyle en C1-C4, alcoxy en C1-C4, alkylthio en C1-C4, le chlore, un groupe cyano, méthylsulfonyle, nitro ou trifluorométhyle et M représente l'hydrogène ou un groupe alkyle en C1-C4, alcoxy en C1-C4, alkylthio en C1-C4, le chlore, un groupe cyano, méthylsulfonyle, nitro ou trifluorométhyle.

3. Dérivés de la saccharine de formule I des revendications 1 et 2, dans laquelle L et M représentent respectivement l'hydrogène, des groupes méthyle, méthoxy, méthylthio, le chlore, des groupes cyano, méthylsulfonyle, nitro ou trifluorométhyle.

4. Produit herbicide contenant au moins un dérivé de la saccharine de formule I de la revendication 1 et des additifs inertes usuels.

5. Procédé pour combattre les croissances de végétaux indésirables, caractérisé par le fait que l'on fait agir une quantité herbicide efficace d'un dérivé de la saccharine de formule I de la revendication 1 sur les végétaux ou leur habitat.

6. Procédé de préparation des composés de formule I de la revendication 1, caractérisé par le fait que l'on acyle les composés de départ de formule II ou III 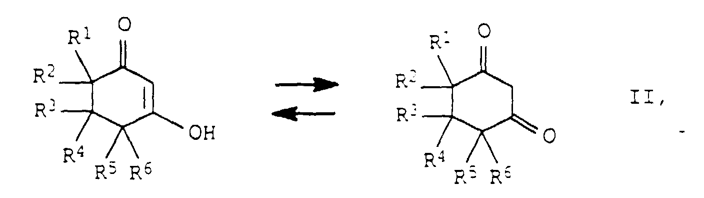 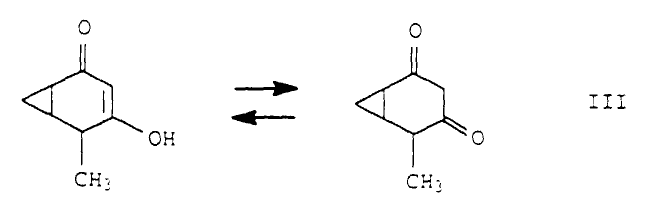 à l'aide d'un chlorure d'acide de formule V. 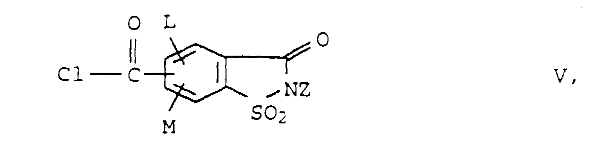 dans laquelle L, M et Z ont les significations indiquées dans la revendication 1, et l'on transpose le produit d'acylation, en présence d'un catalyseur, en les composés I.

7. Dérivés de la saccharine répondant à la formule V 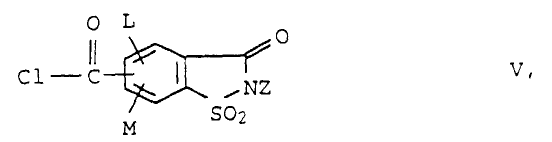 dans laquelle L, M et Z ont les significations suivantes :
L,M : l'hydrogène, des groupes alkyle en C1-C4, alcoxy en C1-C4, alkylthio en C1-C4, le chlore, des groupes cyano, méthylsulfonyle, nitro ou trifluorométhyle ;
Z : l'hydrogène, un groupe alkyle en C1-C4, cycloalkyle en C3-C8, alcényle en C3-C6, alcynyle en C3-C5, acyle en C1-C4, benzyle ou phényle, les cycles phényle pouvant le cas échéant être substitués par des halogènes ou des groupes alkyle en C1-C4.
